(19) Europäisches Patentamt
European Patent Office
Office européen des brevets

(11) Veröffentlichungsnummer: **0 559 002 A1**

(12) # EUROPÄISCHE PATENTANMELDUNG

(21) Anmeldenummer: **93102474.9**

(22) Anmeldetag: **17.02.93**

(51) Int. Cl.5: **C07C 331/12**, C07C 255/30, C07D 317/56, C07D 295/14, C07D 265/30, C07D 233/54, C07C 323/27, A01N 41/12, A01N 37/34

(30) Priorität: **02.03.92 DE 4206528**

(43) Veröffentlichungstag der Anmeldung:
**08.09.93 Patentblatt 93/36**

(84) Benannte Vertragsstaaten:
**AT CH DE DK FR GB IT LI SE**

(71) Anmelder: **BAYER AG**

**D-51368 Leverkusen(DE)**

(72) Erfinder: **Wachtler, Peter, Dr.**
**Morgengraben 4**
**W-5000 Köln 80(DE)**
Erfinder: **Paulus, Wilfried, Dr.**
**Deswatinesstrasse 90**
**W-4150 Krefeld(DE)**
Erfinder: **Ludwig, Georg-Wilhelm, Dr.**
**Kemmerhofstrasse 181**
**W-4150 Krefeld(DE)**

(54) **Cyanalken-Derivate, Verfahren zu ihrer Herstellung und ihre Verwendung als Mikrobizide für den Materialschutz.**

(57) Beschrieben werden neue Cyanalken-Derivate der Formel (I)

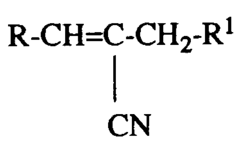

(I)

in welcher R und $R^1$ die in der Beschreibung angegebene Bedeutung haben sowie ein Verfahren zu deren Herstellung.

Die neuen Cyanalken-Derivate werden zur Bekämpfung von Mikroorganismen zum Schutz technischer Materialien verwendet.

EP 0 559 002 A1

Die Erfindung betrifft neue Cyanalken-Derivate, ein Verfahren zu ihrer Herstellung und ihre Verwendung als Mikrobizide für den Materialschutz.

Es ist bereits bekannt, daß die in der EP-A-403 884 beschriebenen Dibrompropanol-Derivate über mikrobizide Eigenschalten verfügen. Diese Verbindungen sind jedoch aufgrund ihrer chemischen Reaktivität im alkalischen Medium, wie z.B. Dispersionsfarben, nicht besonders stabil. Dieser Nachteil wird durch die erfindungsgemäßen Verbindungen vermieden.

Gegenstand der Anmeldung sind daher Cyanalken-Derivate der Formel

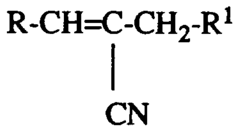

(I)

in der

R für gegebenenfalls substituiertes $C_1$-$C_{12}$-Alkyl, Alkoxyalkyl, Alkylmercaptoalkyl, Cycloalkyl, $C_2$-$C_8$-Alkenyl, Cycloalkenyl, Aryl, Aralkyl, Aryl-alkenyl oder Heteryl

und

$R^1$ für NH-$R^2$,

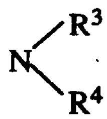

,

S-$R^5$, SO-$R^5$, $SO_2$-$R^5$,

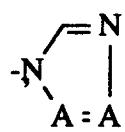

CN, NCS oder SCN steht, wobei

$R^2$ für Alkyl, Aryl oder Aralkyl und

$R^3$, $R^4$ für Alkyl, Aryl oder Aralkyl stehen oder

$R^3$ und $R^4$ einen sechsgliedrigen, durch O oder N unterbrochenen aliphatischen Ring, der durch ein oder zwei Methylgruppen substituiert sein kann, bilden,

$R^5$ Alkyl, Aryl oder Aralkyl bedeutet und

A für CH oder N steht,

ausgenommen Verbindungen, in denen

R für Phenyl steht und

$R^2$ für tert.-Butyl, oder

$R^3$ = $R^4$ für $C_2H_5$ oder $^i$Pro steht oder

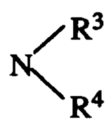

Piperidin oder Morpholin ist.

Alkyl, einzeln oder in zusammengesetzten Resten, steht im folgenden für $C_1$-$C_{12}$-Alkyl, vorzugsweise $C_1$-$C_6$-Alkyl wie Methyl, Ethyl, n-Propyl, i-Propyl, n-Butyl, tert.-Butyl, n-Hexyl, i-Octyl und n-Dodecyl, welches gegebenenfalls durch Halogenatome wie Fluor, Chlor oder Brom oder Hydroxy substituiert ist, z.B. Trifluormethyl oder Trichlormethyl, 2-Methoxymethyl, 2-Ethoxyethyl, 2-Chlorethyl, 2-Trifluormethylmercaptoethyl, 2,3-Dibrompropyl, Hydroxyethyl und Methylthioethyl.

Cycloalkyl steht im folgenden für Cycloalkyl mit 5 bis 7 Kohlenstoffatomen, vorzugsweise für Cyclohexyl und durch Halogen, insbesondere Chloratome und/oder $C_1$-$C_4$-Alkylgruppen substituierte Cyclohexylreste, wie 4-Methylcyclohexyl, 2,6-Dimethylcyclohexyl und 4-tert.-Butylcyclohexyl.

Alkenyl steht im folgenden für Alkenyl mit 2 bis 6 Kohlenstoffatomen, vorzugsweise für gegebenenfalls durch $C_1$-$C_4$-Alkyl substituiertes Allyl, Penten-1-yl oder Penten-2-yl.

Unter dem Begriff Aryl ist unsubstituiertes oder substituiertes Aryl mit vorzugsweise 6 bis 10 C-Atomen im Arylteil zu verstehen. Bevorzugt sind Phenyl und Naphthyl. Die Arylgruppen können 1 bis 3 gleiche oder verschiedene Substituenten aus der Reihe Halogen (insbesondere Chlor, Brom und/oder Fluor), $C_1$-$C_4$-Alkyl, $C_1$-$C_4$-Alkoxy oder $C_1$-$C_4$-Alkylthio, Halogen-$C_1$-$C_2$-alkyl (wie Trifluormethyl, Difluormethyl), Cyano, Nitro, Alkylamino, Alkanoylamino, Arylcarbonylamino, Amino oder Hydroxy enthalten.

Unter Aralkyl sind Kombinationen der oben definierten Arylgruppen mit niedrigen Alkylresten zu verstehen, vorzugsweise der Benzyl- und Phenethylrest.

Unter dem Begriff Hetaryl sind fünf- oder sechsgliedrige, Sauerstoff und/oder 1 bis 2 Stickstoffatome enthaltende, gegebenenfalls substituierte aromatische Ringe, wie z.B. der Pyridyl- oder Furylrest, zu verstehen.

Die erfindungsgemäßen Cyanalken-Derivate werden durch die Formel (I) allgemein definiert.

Bevorzugt sind Verbindungen der Formel (I), in denen

R für gegebenenfalls substituiertes $C_1$-$C_{12}$-Alkyl, $C_5$-$C_7$-Cycloalkyl, Aryl oder Aralkyl und

$R^1$ für CN, NCS, NH-$C_1$-$C_{12}$-Alkyl, Imidazolyl, jeweils gegebenenfalls substituiertes Morpholin oder Thiophenyl oder SCN steht.

Besonders bevorzugt sind solche Verbindungen der Formel (I), in denen

R für jeweils gegebenenfalls substituiertes $C_1$-$C_6$-Alkyl, Cyclohexyl oder Aryl und

$R^1$ für SCN steht.

Ganz besonders bevorzugt sind solche Verbindungen der Formel (I), in denen

R für $C_1$-$C_6$-Alkyl oder gegebenenfalls substituiertes Phenyl und

$R^1$ für SCN steht.

Die erfindungsgemäßen Verbindungen der Formel (I) werden erhalten, indem man Cyanalken-Halogenide der Formel (II)

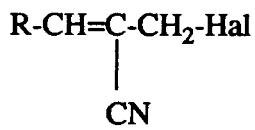

in welcher R die oben genannte Bedeutung hat und Hal für Chlor oder Brom steht mit einem Reaktionspartner der Formel (III) Y-$R^1$ in welcher $R^1$ die oben angegebene Bedeutung hat und Y für Wasserstoff, Natrium, Kalium oder für Trimethylsilyl steht in einem inerten Lösungsmittel umsetzt.

Y = H, Na, K, TMS.

Das als Ausgangsverbindung benötigte Reagenz der allgemeinen Formel (II) wird auf literaturbekanntem Weg (vgl. z.B. J. Org. Chem. 41 (1976), 20, 3241; J. Org. Chem. 42 (1977), 12, 2094; J. Org. Chem. 28 - (1963), 8, 1983; Synth. Commun. 15 (1985), 12, 1067) aus einer Verbindung der allgemeinen Formel (IV) nach folgendem Formelschema gewonnen.

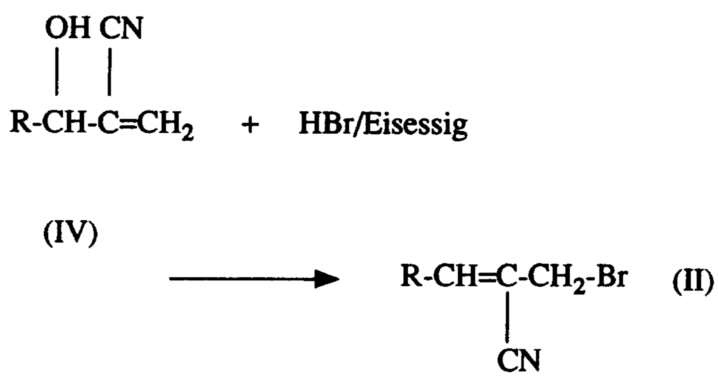

(IV)

(II)

Alternativ hierzu kann auch die entsprechende Chlorverbindung zum Einsatz kommen, die ihrerseits durch Umsetzung der Verbindung (IV) mit Thionylchlorid erhältlich ist:

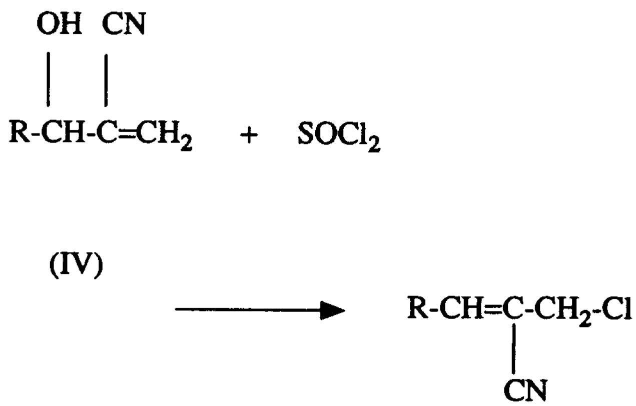

(IV)

Die Acrylnitrilderivate der allgemeinen Formel (IV) sind bekannte Verbindungen und können z.B. wie in der DE-OS 2 155 133 beschrieben, erhalten werden.

Als für die Reaktion geeignete Lösungsmittel seien beispielsweise genannt: Alkohole wie Methanol, Ethanol, Propanol, Ether wie Diethylether, Dioxan, Tetrahydrofuran, Ketone wie Aceton oder Methylethylketon; und DMF, DMSO, Acetonitril, gegebenenfalls in Mischung mit Wasser.

Die Umsetzung wird bei Temperaturen von 0 ° C bis 60 ° C, vorzugsweise bei Temperaturen von 20 bis 40 ° C durchgeführt.

Bei der Durchführung des erfindungsgemäßen Verfahrens setzt man auf 1 Mol der Cyanalkenhalogenidverbindung der Formel (II) 1 bis 1,2 Mol des Reaktionspartners der Formel (III) ein.

Zur Beschleunigung der Reaktion kann es in manchen Fällen vorteilhaft sein, dem Reaktionsgemisch einen Säureakzeptor zuzusetzen. Hierfür sind Basen wie beispielsweise Pyridin, Collidin oder Triethylamin geeignet.

Die Aufarbeitung und Isolierung der Cyanalken-Verbindungen der Formel (I) erfolgt nach bekannten Methoden und wird in den Beispielen näher erläutert.

Unter den erfindungsgemäßen Verbindungen der allgemeinen Formel (I) sind die Isomerengemische (Z/E-Form) als auch die jeweiligen reinen E- oder Z-Isomeren zu verstehen.

Die erfindungsgemäßen Wirkstoffe bzw. Mittel weisen eine starke Wirkung gegen Mikroorganismen auf. Sie werden im Materialschutz zum Schutz technischer Materialien verwendet: sie sind vor allem wirksam gegen Schimmelpilze, holzverfärbende und holzzerstörende Pilze und Bakterien, sowie gegen Hefen, Algen, Organismen die Unterwasseranstriche besiedeln und Schleimorganismen. Beispielhaft - ohne jedoch zu limitieren - seien die folgenden Gattungen von Mikroorganismen genannt:

Alternaria wie Alternaria tenuis, Aspergillus wie Aspergillus niger und Aspergillus terreus, Aureobasidium wie Aureobasidium pullulans, Chaetomium wie Chaetomium globosum, Cladosporium wie Cladosporium herbarum, Coniophora wie Coniophora puteana, Gliocladium wie Gliocladium virens, Lentinus wie Lentinus tigrinus, Paecilomyces wie Paecilomyces varioti, Penicillium wie Penicillium brevicaule, Penicillium glaucum und Penicillium pinophilum, Polyporus wie Polyporus versicolor, Sclerophoma wie Sclerophoma pityophila, Streptoverticillium wie Streptoverticillium reticulum, Trichoderma wie Trichoderma viride, Trichophyton wie Trichophyton mentagrophytes;

Escherichia wie Escherichia coli, Pseudomonas wie Pseudomonas aeruginosa, Staphylococcus wie Staphylococcus aureus;

Candida wie Candida albicans.

Enteremorpha wie Enteromorpha intestinalis

Die Menge der eingesetzten Wirkstoffe ist von der Art und dem Vorkommen der Mikroorganismen der Keimzahl und von dem Medium abhängig. Die optimale Einsatzmenge kann bei der Anwendung jeweils durch Testreihen ermittelt werden. Im allgemeinen ist es jedoch ausreichend 0,001 bis 20 Gew.-%, vorzugsweise 0,05 bis 10 Gew.-%, der Wirkstoffgemische, bezogen auf das zu schützende Material, einzusetzen.

Die neuen Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit Lösungs- bzw. Verdünnungsmitteln, Emulgatoren, Dispergatoren und/oder Binde- oder Fixiermitteln, gegebenenfalls Sikkative und UV-Stabilisatoren und gegebenenfalls Farbstoffen und Pigmenten sowie weiteren Verarbeitungshilfsmitteln.

Als Lösungs- bzw. Verdünnungsmittel kommen organisch-chemische Lösungsmittel oder Lösungsmittelgemische und/oder ein polares organisches Lösungsmittel oder Lösungsmittelgemische und/oder ein öliges bzw. ölartiges organisch-chemisches Lösungsmittel oder Lösungsmittelgemisch und/oder Wasser mit vorzugsweise einem Emulgator und/oder Netzmittel in Frage. Als übliche schwerflüchtige, wasserunlösliche ölige oder ölartige Lösungsmittel werden vorzugsweise die jeweiligen Mineralöle/mineralölhaltige Lösungsmittelgemische oder deren Aromatenfraktionen verwendet. Beispielhaft seien Testbenzin, Petroleum oder Alkylbenzole genannt, daneben Spindelöl und Monochlornaphthalin. Die Siedebereiche dieser schwerflüchtigen Lösemittel(gemische) überstreichen den Bereich von ca 170 °C bis maximal 350 °C.

Die vorbeschriebenen schwerflüchtigen öligen oder ölartigen Lösungsmittel können teilweise durch leichter flüchtige organisch-chemische Lösungsmittel ersetzt werden.

Zur Herstellung eines Holzschutzmittels wird vorzugsweise ein Teil des oben beschriebenen Lösungsmittels oder Lösungsmittelgemisches durch ein polares organisch-chemisches Lösungsmittel oder Lösungsmittelgemisches ersetzt. Vorzugsweise gelangen dabei Lösungsmittel, die Hydroxygruppen, Estergruppen, Ethergruppen oder Gemische dieser Funktionalität enthalten, zum Einsatz. Beispielhaft seien Ester oder Glykolether genannt. Als Bindemittel werden erfindungsgemäß verstanden wasserverdünnbare bzw. in organisch-chemischen Lösungsmitteln löslich, dispergier- oder emulgierbare Kunstharze, bindende trocknende Öle, z.B. auf Basis von Acrylharzen, Vinylharzen, Polyesterharzen, Polyurethanharzen, Alkydharzen, Phenolharzen, Kohlenwasserstoffharzen, Silikonharzen. Das benutzte Bindemittel kann als Lösung, Emulsion oder Dispersion eingesetzt werden. Vorzugsweise werden Gemische aus Alkydharzen und trocknendem pflanzlichen Öl verwendet. Besonders bevorzugt sind Alkydharze mit einem Ölanteil zwischen 45 und 70 %.

Das erwähnte Bindemittel kann ganz oder teilweise durch ein Fixierungsmittelgemisch oder ein Weichmachergemisch ersetzt werden. Diese Zusätze sollen einer Verflüchtigung der Wirkstoffe sowie einer Kristallisation bzw. eines Ausfällens vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Burylstearat und Amylstearat, Oleate wie Buryloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylbenzophenon.

Als Lösungs- bzw. Verdünnungsmittel kommt vorzugsweise Wasser in Frage, gegebenenfalls in Mischung mit einem oder mehreren der obengenannten Lösungs- bzw. Verdünnungsmittel, Emulgatoren und Dispergatoren.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichimittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Bevorzugte technische Materialien im Sinne der Erfindung sind Klebstoffe, Leime, Papiere und Kartone, Leder, Holz, Anstrichmittel, Kühlschmiermittel, wäßrige Hydraulikflüssigkeiten und Kühlkreisläufe und allgemein wäßrige funktionelle Flüssigkeiten.

Die Wirksamkeit und das Wirkungsspektrum der erfindungsgemäßen Wirkstoffe bzw. den daraus herstellbaren Mitteln, Konzentraten oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

In vielen Fällen erhält man dabei synergistische Effekte, d.h. die Wirksamkeit der Mischung ist größer als die der Wirksamkeit der Einzelkomponenten. Besonders günstige Mischungspartner sind z.B. die folgenden Verbindungen:
Sulfenamide wie Dichlofluanid (Euparen), Tolylfluanid (Methyleuparen), Folpet, Fluorfolpet;
Benzimidazole wie Carbendazim (MBC), Benomyl, Fuberidazole, Thiabendazole oder deren Salze;

Thiocyanate wie Thiocyanatomethylthiobenzothiazol (TCMTB), Methylenbisthiocyanat (MBT);
quartäre Ammoniumverbindungen wie Benzyldimethyltetradecylammoniumchlorid, Benzyl-dimethyl-dodecyl-ammoniumchlorid, Didecyl-dimethyl-ammoniumchlorid;
Morpholinderivate wie $C_{11}$-$C_{14}$-4-Alkyl-2,6-dimethyl-morpholin-homologe(Tridemorph),(±)-cis-4-[3-tert-Butylphenyl)-2-methylpropyl]-2,6-dimethylmorpholin (Fenpropimorph), Falimorph;
Phenole wie o-Phenylphenol, Tribromphenol, Tetrachlorphenol, Pentachlorphenol, 3-Methyl-4-chlorphenol, Dichlorophen, Chlorophen oder deren Salze;
Azole wie Triadimefon, Triadimenol, Bitertanol, Tebuconazole, Propiconazole, Azaconazole, Hexaconazole, Prochloraz;
Iodpropargylderivate wie Iodpropargyl-butylcarbamat (IPBC), -chlorophenylformal, -phenylcarbamat, -hexylcarbamat, -cyclohexylcarbamat, Iodpropargyloxyethyl-phenylcarbamat;
Iodderivate wie Diiodmethyl-p-arylsulfone z.B. Diiodmethyl-p-tolylsulfon;
Bromderivate wie Bronopol und Bronidox;
Isothiazolinone wie N-Methylisothiazolin-3-on, 5-Chlor-N-methylisothiazolin-3-on, 4,5-Dichloro-N-octylisothiazolin-3-on, N-Octylisothiazolin-3-on (Octhilinone);
Benzisothiazolinone, Cyclopentenisothiazoline;
Pyridine wie 1-Hydroxy-2-pyridinthion (und ihre Na-, Fe-, Mn, Zn-Salze), Tetrachlor-4-methylsulphonylpyridin;
Metallseifen wie Zinn-, Kupfer-, Zink-naphthenat, -oc-toat, -2-ethylhexanoat, -oleat, phosphat, -benzoat,
Oxide wie TBTO, $Cu_2O$, CuO, ZnO;
Organische Zinnverbindungen wie Tributylzinnnaphthenat und Tributylzinnoxid;
Dialkyldithiocarbamate wie Na- und Zn-Salze von Dialkyldithiocarbamaten, Tetramethyldiuramdisulfid (TMTD);
Nitrile wie 2,4,5,6-Tetrachlorisophthalonitril (Chlorthalonil) u.a Mikrobizide mit aktivierten Halogengruppen wie Cl-Ac, MCA, Tectamer:
Benzthiazole wie 2-Mercaptobenzothiazol;
Chinoline wie 8-Hydroxychinolin;
Formaldehydabspaltende Verbindungen wie Benzylalkohol-mono(poly)hemiformal, Oxazolidine, Hexahydro-s-triazine, N-Methylolchloracetamid;
Tris-N-(Cyclohexyldiazeniumdioxy)-Aluminium N-(Cyclohexyldiazeniumdioxy)-Tributylzinn bzw. deren Kalium-Salze, Bis-(N-cyclohexyldiazeniumdioxy)-Kupfer oder Aluminium).

Als Insektizide werden bevorzugt zugesetzt:
Phosphorsäureester wie Azinphos-ethyl, Azinphos-methyl, 1-(4-Chlorphenyl)-4-(O-ethyl, S-propyl)-phosphoryloxypyrazol (TIA-230), Chlorpyrifos, Coumaphos, Demeton, Demeton-S-methyl, Diazinon, Dichlorvos, Dimethoate, Ethoprophos, Etrimfos, Fenitrothion, Fention, Heptenophos, Parathion, Parathion-methyl, Phosalone, Phoxion, Pirimiphos-ethyl, Pirimiphos-methyl, Profenofos, Prothiofos, Sulprofos, Triazophos und Trichlorphon.

Carbamate wie Aldicarb, Bendiocarb, BPMC (2-(1-Methylpropyl)phenylmethylcarbamat), Butocarboxim, Butoxycarboxim, Carbaryl, Carbofuran, Carbosulfan, Cloethocarb, Isoprocarb, Methomyl, Oxamyl, Pirmicarb, Promecarb, Propoxur und Thiodicarb.

Pyrethroide wie Allethrin, Alphamethrin, Bioresmethrin, Byfenthrin (FMC 54 800), Cycloprothrin, Cyfluthrin, Decamethrion, Cyhalothrin, Cypermethrin, Deltamethrin, Alpha-cyano-3-phenyl-2-methylbenzyl-2,2-dimethyl-3-(2-chlor-2-trifluormethylvinyl)cyclopropancarboxylat, Fenpropathrin, Fenfluthrin, Fenvalerate, Flucythrinate, Flumethrin, Fluvalinate, Permethrin und Resmethrin; Nitroimide wie 1-[(6-Chlor-3-pyridinyl)-methyl]-4,5-dihydro-N-nitro-1H-imidazol-2-anin (Imidacloprid).

Organosiliciumverbindungen, vorzugsweise Dimethyl(phenyl)silylmethyl-3-phenoxy-benzylether wie z.B. Dimethyl(4-ethoxyphenyl)-silylmethyl-3-phenoxybenzylether oder Dimethyl(phenyl)-silylmethyl-2-phenoxy-6-pyridylmethylether wie z.B. Dimethyl(9-ethoxyphenyl)-silylmethyl-2-phenoxy-6-pyridylmethylether oder (Phenyl)[3-(3-phenoxyphenyl)-propyl](dimethyl)-silane wie z.B. (4-ethoxyphenyl)-[3-(4-fluoro-3-phenoxyphenyl)-propyl]dimethyl-silan.

Als andere Wirkstoffe kommen in Betracht Algizide, Molluskizide, Wirkstoffe gegen "sea animals", die sich auf z.B. Schiffsbodenanstrichen ansiedeln.

Die zum Schutz der technischen Materialien verwendeten mikrobiziden Mittel oder Konzentrate enthalten die erfindungsgemäßen Wirkstoffe in einer Konzentration von 0,01 bis 95 Gew.-%, insbesondere 0,01 bis 60 Gew.-%, daneben gegebenenfalls 0,001 bis 10 Gew.-% eines geeigneten weiteren Fungizids, Insektizids oder eines weiteren Wirkstoffs wie oben genannt.

Die erfindungsgemäßen Wirkstoffe bzw. Mittel ermöglichen in vorteilhafter Weise, die bisher verfügbaren mikrobiziden Mittel durch effektivere zu ersetzen. Sie zeigen eine gute Stabilität und haben in

vorteilhafter Weise ein breites Wirkungsspektrum.

Die nachfolgenden Beispiele dienen zur Erläuterung der Erfindung ohne sie darauf zu limitieren. Teile und Prozentangaben bedeuten Gewichtsteile bzw. Gewichtsprozente.

Beispiel 1

26,1 g (0,15 Mol) 1-Ethyl-2-cyano-3-brom-propen werden in 100 ml Acetonitril gelöst vorgelegt und bei 20 °C mit 14,6 g (0,15 Mol) Kaliumrhodanid versetzt. Nach 5 Stunden Reaktionsdauer bei 20 °C ist die Umsetzung abgeschlossen. Der Kolbeninhalt wird dann in 500 ml Wasser eingerührt und das Produkt durch Extraktion mit Methylenchlorid isoliert. Mach Trocknen über $Na_2SO_4$ und Einengen am Rotations-Verdampfer verbleiben 20,5 g eines orange-gefärbten Öls von 1-Ethyl-2-cyano-3-thiocyanato-propen. Die Verbindung wird durch ihr $^1$H-NMR-Spektrum ($\delta$ (H-Olefin) = 6,49 ppm) charakterisiert.

Beispiel 2

15,0 g (0,065 Mol) 1-n-Hexyl-2-cyano-3-brom-propen werden zusammen mit 6,6 g (0,065 Mol) n-Hexylamin in 100 ml Tetrahydrofuran bei Raumtemperatur vorgelegt. Unter Rühren werden 6,6 g (0,065 Mol) Triethylamin zugefügt und das erhaltene Gemisch dann 10 Stunden bei Raumtemperatur gerührt; anschließend wird in 400 ml Wasser eingerührt, mit Methylenchlorid extrahiert und im Vakuum das Lösungsmittel entfernt. Es verbleiben 13,2 g eines hellgelb gefärbten Öles von 1-n-Hexyl-2-cyano-3-n-hexylamino-propen. Die Verbindung wird durch ihr $^1$H-NMR-Spektrum ($\delta$ (H-Olefin) = 6,30 ppm) charakterisiert.

In analoger Weise und gemäß den allgemeinen Angaben in der Beschreibung werden die folgenden Verbindungen der Formel (I)

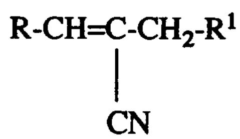

(I)

hergestellt:

<u>Tabelle 1</u>

| Beispiel | R | $R^1$ | Physikalische Daten |
|---|---|---|---|
| 3 | n-$C_3H_7$ | SCN | δ(CH=) : 6,54 ppm (t) |
| 4 | n-$C_4H_9$ | SCN | δ(CH=) : 6,54 ppm (t) |
| 5 | i-$C_4H_9$ | SCN | δ(CH=) : 6,55 ppm (t) |
| 6 | s-$C_4H_9$ | SCN | δ(CH=) : 6,30 ppm (d) |
| 7 | n-$C_6H_{13}$ | SCN | δ(CH=) : 6,54 ppm (t) |
| 8 | n-$C_{10}H_{21}$ | SCN | δ(CH=) : 6,54 ppm (t) |
| 9 | 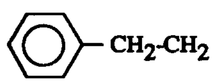 | SCN | δ(CH=) : 6,50 ppm (t) |
| 10 | 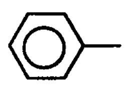 | SCN | δ(CH=) : 7,21 ppm (s) |
| 11 | 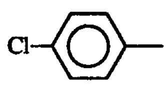 | SCN | δ(CH=) : 7,16 ppm (s) |
| 12 | 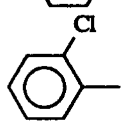 | SCN | δ(CH=) : 7,59 ppm (s) |
| 13 | 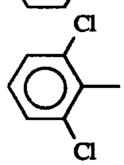 | SCN | δ(CH=) : 7,40 ppm (s) |
| 14 | 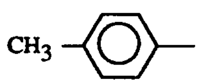 | SCN | δ(CH=) : 7,16 ppm (s) |
| 15 | 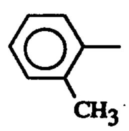 | SCN | δ(CH=) : 7,44 ppm (s) |
| 16 | 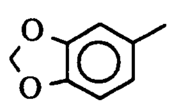 | SCN | δ(CH=) : 7,08 ppm (s) |
| 17 | 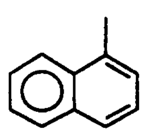 | SCN | IR: CN 2225 $cm^{-1}$<br>SCN 2160 $cm^{-1}$ |

Tabelle 2

| Beispiel | R | $R^1$ | Physikalische Daten |
|---|---|---|---|
| 18 | $n\text{-}C_3H_7$ | $NH\text{-}n\text{-}C_6H_{13}$ | δ(CH=) : 6,30 ppm (t) |
| 19 | $n\text{-}C_4H_9$ | 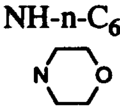 | δ(CH=) : 6,33 ppm (t) |
| 20 | $n\text{-}C_6H_{13}$ | 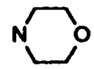 | δ(CH=) : 6,33 ppm (t) |
| 21 | $n\text{-}C_8H_{17}$ | 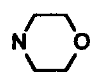 | δ(CH=) : 6,33 ppm (t) |
| 22 | $n\text{-}C_{10}H_{21}$ | 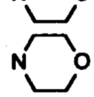 | δ(CH=) : 6,33 ppm (t) |
| 23 | $n\text{-}C_4H_9$ | 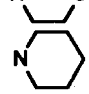 | δ(CH=) : 6,29 ppm (t) |
| 24 | $n\text{-}C_6H_{13}$ | 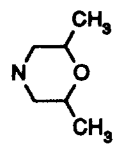 | δ(CH=) : 6,30 ppm (t) |
| 25 | $n\text{-}C_8H_{17}$ | 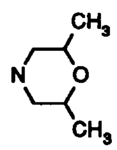 | δ(CH=) : 6,29 ppm (t) |
| 26 | $n\text{-}C_{10}H_{21}$ | 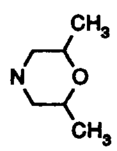 | δ(CH=) : 6,30 ppm (t) |
| 27 | 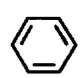 | 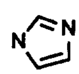 | δ(CH=) : 7,08 ppm (s) |
| 28 | 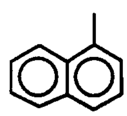 | 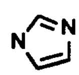 | Schmp. = 167-69°C |
| 29 | $C_3H_7$ | 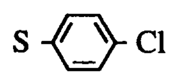 | δ(CH=) : 6,00 ppm (t) |
| 30 | $n\text{-}C_6H_{13}$ | 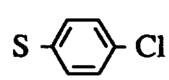 | δ(CH=) : 5,99 ppm (t) |

(s) = Singulett; (t) = Triplett

Anwendungsbeispiel

A. Zum Nachweis der Wirksamkeit gegen Pilze werden die minimalen Hemm-Konzentrationen (MHK) von erfindungsgemäßen Mitteln bestimmt:

Ein Agar, der aus Bierwürze und Pepton hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle 1 aufgeführten Testorganismen. Nach 2-wöchiger Lagerung bei 28 °C und 60 bis 70 % rel. Luftfeuchtigkeit wird die MHK bestimmt. MHK ist die niedrigste Konzentration an Wirkstoff,

bei der keinerlei Bewuchs durch die verwendete Mikrobenart erfolgt, sie ist in der nachstehenden Tabelle 1 angegeben.

**Tabelle I:** MHK's [mg/l] bei der Einwirkung erfindungsgemäßer Cyanalkan-Derivate auf Mikroorganismen

| Testorganismen | Beispiele | | | | | | | | |
|---|---|---|---|---|---|---|---|---|---|
| | 1 | 3 | 4 | 5 | 6 | 7 | 9 | 10 | 15 |
| Penicillium brevicaule | 50 | 100 | 20 | 50 | 50 | 20 | 20 | 75 | 50 |
| Chaetomium globosum | 100 | 400 | 50 | 50 | 100 | 15 | 20 | 75 | 75 |
| Aspergillus niger | 100 | 400 | 200 | 400 | 800 | 75 | 400 | 100 | 50 |
| Lentinus tigrinus | 15 | 100 | 20 | 50 | 50 | 20 | 10 | 20 | 75 |
| Sclerophoma pityophila | 5 | 20 | 5 | 10 | 15 | 50 | 5 | 1 | 3 |
| Trichoderma viride | 250 | 300 | 150 | 200 | 400 | 400 | 200 | 100 | 400 |
| Cladosporium herbarum | 75 | 600 | 100 | 200 | 400 | 200 | 200 | 100 | 300 |
| Alternaria tenius | 35 | 200 | 20 | 75 | 200 | 200 | 100 | 50 | 100 |
| Aureobasidium pullulans | 35 | 200 | 35 | 100 | 200 | 50 | 50 | 20 | 150 |
| Staphylococcus aureus | 400 | >800 | 100 | 100 | 100 | <50 | 100 | 50 | >80 |

## Patentansprüche

**1.** Cyanalken-Derivate der Formel

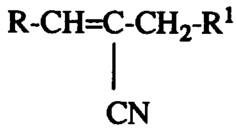

(I)

in der

R für gegebenenfalls substituiertes $C_1$-$C_{12}$-Alkyl, Alkoxyalkyl, Alkylmercaptoalkyl, Cycloalkyl, $C_2$-$C_8$-Alkenyl, Cycloalkenyl, Aryl, Aralkyl, Aryl-alkenyl oder Hetaryl

und

$R^1$ für

NH-$R^2$,

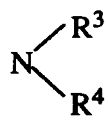

,

S-$R^5$, SO-$R^5$, $SO_2$-$R^5$,

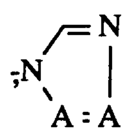

CN, NCS oder SCN steht, wobei

$R^2$ für Alkyl, Aryl oder Aralkyl und

$R^3$, $R^4$ für Alkyl, Aryl oder Aralkyl stehen oder

$R^3$ und $R^4$ einen sechsgliedrigen, durch O oder N unterbrochenen aliphatischen Ring, der durch ein oder zwei Methylgruppen substituiert sein kann, bilden,

$R^5$ Alkyl, Alyl oder Aralkyl bedeutet und

A für CH oder N steht,

ausgenommen Verbindungen, in denen

R für Phenyl steht und

$R^2$ für tert.-Butyl, oder

$R^3$ = $R^4$ für $C_2H_5$ oder $^{i}$Pro steht oder

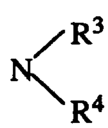

= Piperidin oder Morpholin ist.

**2.** Verbindungen der Formel (I) gemäß Anspruch 1, in denen

R für gegebenenfalls substituiertes $C_1$-$C_{12}$-Alkyl, $C_5$-$C_7$-Cycloalkyl, Aryl oder Aralkyl und

$R^1$ für CN, NCS, NH-$C_1$-$C_{12}$-Alkyl, Imidazolyl, jeweils gegebenenfalls substituiertes Morpholin oder Thiophenyl oder SCN steht.

3. Verbindungen der Formel (I) gemäß Anspruch 1, in denen
   R für jeweils gegebenenfalls substituiertes $C_1$-$C_6$-Alkyl, Cyclohexyl oder Aryl und
   $R^1$ für SCN steht.

4. Mikrobizide Mittel, gekennzeichnet durch einen Gehalt an mindestens einem Cyanalken-Derivat der Formel (I) nach Anspruch 1.

5. Verfahren zur Bekämpfung von Mikroorganismen, dadurch gekennzeichnet, daß man Cyanalken-Derivate der Formel (I) nach Anspruch 1 auf technische Materialien einwirken läßt oder diese mit den Cyanalken-Derivaten versetzt.

6. Verfahren zur Herstellung von Cyanalken-Derivaten der Formel (I)

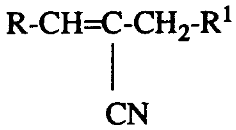

(I)

in welcher R und $R^1$ die in Anspruch 1 angegebene Bedeutung haben, dadurch gekennzeichnet, daß man Cyanalken-Halogenide der Formel (II)

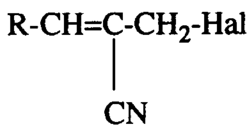

(II)

in welcher R die oben genannte Bedeutung hat und Hal für Chlor oder Brom steht mit Verbindungen der Formel (III)

$Y\text{-}R^1$ (III)

in welcher $R^1$ die oben genannte Bedeutung hat und Y für Wasserstoff, Natrium, Kalium oder Trimethylsilyl steht, in einem inerten Lösungsmittel umsetzt.

7. Verwendung der Cyanalken-Derivate der Formel (I) nach den Ansprüchen 1 bis 6 zur Bekämpfung von Mikroorganismen zum Schutz technischer Materialien.

Europäisches Patentamt

# EUROPÄISCHER RECHERCHENBERICHT

Nummer der Anmeldung
EP 93 10 2474
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,A | EP-A-0 403 884 (BAYER)<br>* Seite 2 *<br>--- | 1,4,5,7 | C07C331/12<br>C07C255/30<br>C07D317/56<br>C07D295/14<br>C07D265/30<br>C07D233/54<br>C07C323/27<br>A01N41/12<br>A01N37/34 |
| X | CHEMICAL ABSTRACTS, vol. 73, no. 3, 20. Juli 1970, Columbus, Ohio, US; abstract no. 14120h, K.N. ZELENIN, et al.: 'Dialkylhydrazones of alpha-alkyl-beta-chloroacroleins and their conversion to dialkyl aminoacrylonitriles'<br>Seite 304 ;<br>& DOKL. AKAD. NAUK. SSSR, 1970, 190(6), 1354-7<br>--- | 1 | |
| X | CHEMICAL ABSTRACTS, vol. 76, no. 3, 17. Januar 1972, Columbus, Ohio, US; abstract no. 14144f,<br>Seite 351 ;<br>& JP-A-46 037 572 (TORAY INDUSTRIES) 5. November 1971<br>* Zusammenfassung *<br>--- | 1 | **RECHERCHIERTE SACHGEBIETE (Int. Cl.5 )** |
| X | FR-A-2 094 335 (RHÔNE-POULENC)<br>* Seite 5, Zeile 5; Seite 6, Zeilen 20-21 *<br>--- | 1,2 | C07C<br>C07D |
| X | US-A-3 391 050 (R.M. CRESSWELL et al.)<br>* Beispiele 43-48 *<br>--- | 1,2 | |
| X | BULLETIN DE LA SOCIETE CHIMIQUE DE FRANCE, Nr. 9-10, September 1974, Paris, FR, Seiten 2065 - 2071<br>* Tabelle I *<br>---<br>-/-- | 1,2 | |

Der vorliegende Recherchenbericht wurde für alle Patentansprüche erstellt

| Recherchenort | Abschlußdatum der Recherche | Prüfer |
|---|---|---|
| DEN HAAG | 02 JUNI 1993 | RUSSELL F. ENGLISH |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 93 10 2474
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | CHEMICAL ABSTRACTS, vol. 95, no. 5, 3. August 1981, Columbus, Ohio, US; abstract no. 42590j, K. POPANDOVA-YAMBOLIEVA, et al.: 'Reaction of 3-(dimethylamino)propionitrile with aromatic aldehydes' Seite 713 ; & GOD. SOFII. UNIV., KHIM. FAK., 1980, 71(2), 73-79 --- | 1 | |
| X | US-A-4 258 045 (M. POE et al.) * Beispiel 1, Schritt A * --- | 1 | |
| X | EP-A-0 237 516 (ROUSSELOT) * Beispiel 22b * --- | 1 | |
| X | EP-A-0 266 549 (BOEHRINGER BIOCHEMIA ROBIN) * Seite 3; Beispiel 13 * --- | 1,2 | |
| X | JOURNAL OF MEDICINAL CHEMISTRY, Bd. 32, Nr. 8, August 1989, Washington, DC, US, Seiten 1895 - 1905 C. DIAS SELASSIE, ET AL.: 'On the structure selectivity problem in drug design. A comparative study of benzylpyrimidine inhibition of vertebrate and bacterial dihydrofolate reductase via molecular graphics and quantitative structure-activity relationships' * Seite 1897, linke Spalte, Zeile 16 * --- -/-- | 1 | RECHERCHIERTE SACHGEBIETE (Int. Cl.5 ) |

Der vorliegende Recherchenbericht wurde für alle Patentansprüche erstellt

| Recherchenort | Abschlußdatum der Recherche | Prüfer |
|---|---|---|
| DEN HAAG | 02 JUNI 1993 | RUSSELL F. ENGLISH |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 93 10 2474
Seite 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | JOURNAL OF ORGANIC CHEMISTRY,<br>Bd. 41, Nr. 20, 1. Oktober 1976,<br>Washington, DC, US,<br>Seiten 3241 - 3245<br>N.H. CROMWELL, ET AL.: 'Mobile activated allyl systems. 19. Reactions of amines with alpha-(bromomethyl)cinnamonitrile'<br>* Verbindungen 3a-e *<br>----- | 1,2 | |

RECHERCHIERTE SACHGEBIETE (Int. Cl.5 )

Der vorliegende Recherchenbericht wurde für alle Patentansprüche erstellt

| Recherchenort | Abschlußdatum der Recherche | Prüfer |
|---|---|---|
| DEN HAAG | 02 JUNI 1993 | RUSSELL F. ENGLISH |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)